# NEW EUROPEAN PATENT SPECIFICATION

(11) **EP 0 627 266 B2**
(45) Date of publication and mention of the opposition decision: **27.12.2006**
(45) Mention of the grant of the patent: 25.08.1999
(21) Application number: 94304001.4
(22) Date of filing: 03.06.1994
(51) Int. Cl.: B05B 7/00, A61M 11/06

(54) **Nebuliser**
Vernebler
Nébuliseur

(30) Priority: 04.06.1993 GB 9311614
(43) Date of publication of application: 07.12.1994
(73) Proprietor: Medic-Aid Limited, Bognor Regis, West Sussex PO22 9SL (GB)
(72) Inventor: Denyer, John Stanley Harold, Chichester, West Sussex (GB)
(74) Representative: Wright, Howard Hugh Burnby

(56) References cited:
- EP-A- 0 052 284
- EP-A- 0 261 649
- EP-A- 0 471 323
- DE-A- 849 172
- DE-A- 927 920
- DE-A- 3 429 411
- FR-A- 2 694 500
- GB-A- 838 453
- US-A- 4 746 067
- Prospekt Medic-Aid Sidestream, 05/91

## Description

The present invention relates to atomizers and nozzles for atomizers used for atomising fluids or powders carried in a stream of pressurised gas. A nebulizer having the features of the preamble of claim 1 is known from GB-A-838 453. The invention is particularly useful for introducing a medicament in the form of a medicinal fluid or powder into gas for inhalation by a patient, thereby administering a medicine to the patient. The substance may typically be a powder, a liquid or a particulate suspension. German Patent Specification DE 34 29 411 describes a nozzle for atomising, distributing and mixing fluid or powdery substances by means of a stream of gas and, in particular for producing aerosols for the purposes of inhalation. More specifically claim 1 of DE 34 29 411 claims such as a nozzle consisting of a nozzle body and an airstream control device which can be mounted on the nozzle body, wherein the stream of pressurised gas emerging centrally from the head of the nozzle through a pressurised gas duct draws in the material to be atomised from two intake ducts which are adjacent the pressurized gas ducts and are diametrically opposite one another, and wherein the airstream control device comprises a rebound bar in the outlet cone of the pressurized gas which bar is connected to a ring which can be mounted on the head of the nozzle body and is characterized in that the rebound bar (11) is shaped as a wedge in a manner known per se on its side opposite the nozzle opening (29);
- in that two parallel guide surfaces (15) are provided on the ring (14) and corresponding guide surfaces (17) which are adapted to the first guide surfaces and are aligned therewith are provided on the nozzle head (16) in such a way that, when the airstream control device is mounted, the rebound bar (11) extends transversely to the connection line between the outlet openings (30, 31) of the intake ducts (5, 6); and
- that the outer contour of the ring (14) of the airstream control, device (3) projects over the edge of the nozzle head (16) on all sides.

For reference, copies of Figures 1, 2 and 3 of DE 3429411 are appended to this specification indicating those components mentioned above and bearing reference numbers (11), (29), (15), (14), (17), (16), (30, (31), (5), (6) and (3).
Accurate alignment of the rebound bar is vital to the correct function of such a conventional atomizer as described in DE 34 29 411.

There are several disadvantages to the atomizers that have been used hitherto. The atomizers are often used by elderly or infirm people who must occasionally take the atomizer apart for cleaning. When reassembling the atomizer, the person must take care to locate the ring or tube correctly so that the rebound bar is in correct alignment as indicated above. There are usually only one or two correct orientations which will permit the device to function properly. If the atomizer is incorrectly assembled then it will not function correctly, since the bar will not correctly divert the stream of gas from the nozzle head.

Our investigations have shown that it is unnecessary to form parallel guide surfaces (15) on the ring (14) and corresponding guide surfaces (17) on the nozzle head (16) for the purpose of positioning the rebound bar (11) transversely to the connection line between the outlet openings (30, 31) of the intake ducts (5, 6).

Such a structure complicates production of moulding tools and increases not only the cost of the tooling but also the cost of the finished product.

According to the present invention, a nebuliser for atomising a substance into a stream of gas includes the features of claim 1.

Preferably the angularly displaceable airflow control device includes a tubular carrier which fits on and around the nozzle head to support the bridge bar and which further includes spacing means for positioning the bridge bar in a spaced relationship relative to the reservoir and said at least one gas outlet.

It is advantageous to have as many holes leading to the reservoir as possible. This improves the flow of the substance to the reservoir. Four holes have been found to give a satisfactory flow of the substance whilst keeping the manufacturing cost down.

Preferably the nebuliser includes a source of the substance to be atomised in the form of a container.

It will be appreciated that where the reservoir is annular, the angular position of the diametrically supported rebound bar is irrelevant since it is always disposed diametrically across the reservoir and in line with the pressurized gas outlet.

Embodiments of the present invention will be described hereinafter by way of example only with reference to the drawings in which:

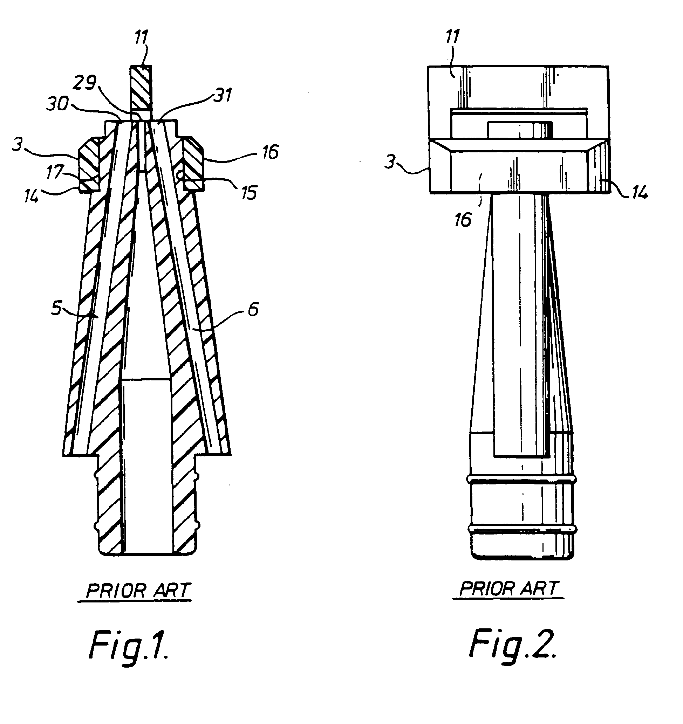

Figure 1 shows a sectional view of a nozzle head according to the prior art;
Figure 2 shows another view of the prior art;
Figure 3 shows a perspective view of the prior art; Figure 4 shows an atomizer of the present invention all of which is shown in exploded cross-section except the nozzle head which is shown unsectioned;
Figure 5 shows a part cross-sectional - part perspective view of the atomizer according to the present invention;
Figure 6 shows a sectional view through the baffle tube of the present invention;
Figure 7 shows a plan view of the nozzle head of the atomizer according to the present invention;
Figure 8 shows an external view of the nozzle head before being moulded into the gas duct;
Figure 9 shows a plan view of a baffle with two portions removed;
Figure 10 shows a side view of the baffle with two portions removed from the mushroom portions;
Figure 11 shows a sectional side view of the baffle assembly and base portion, and
Figure 12 shows a sectional view of the nebuliser in its assembled state.

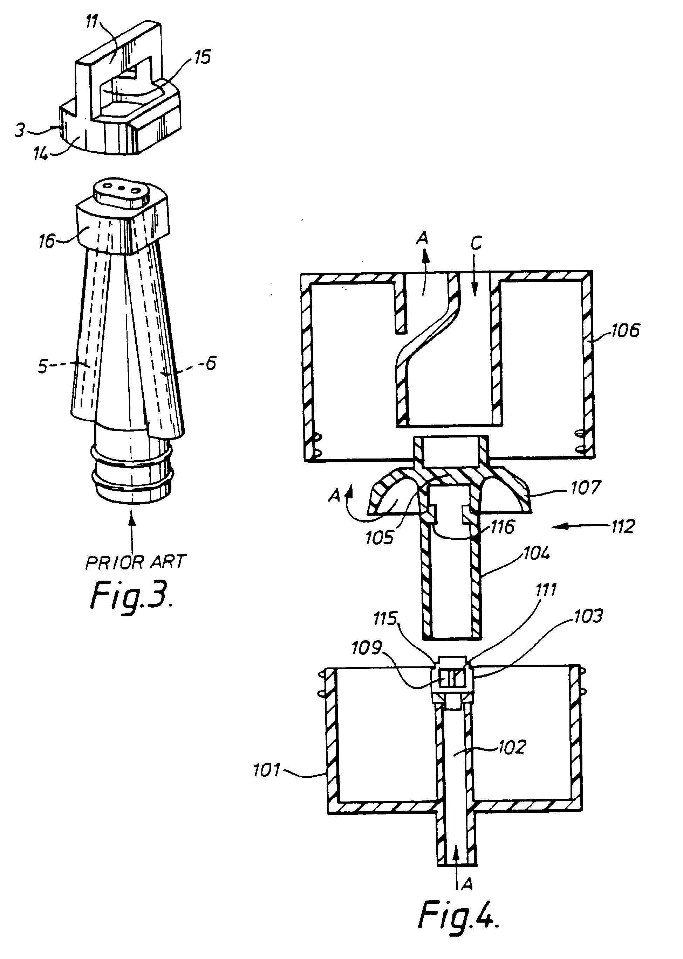

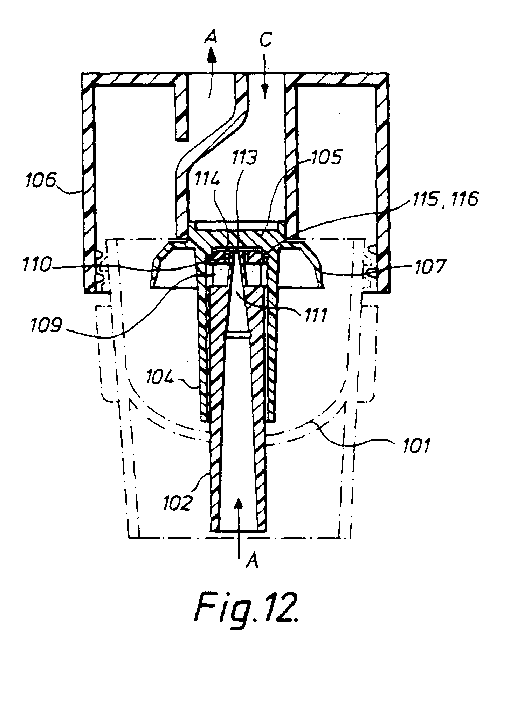

Figures 4 and 12 show an atomizer in which a gas duct (102) leads gas into the atomizer through a primary container (101). The container (101) contains a substance to be atomised into the gas. A lid (106) fits onto the container (101) in an airtight manner. In this embodiment, the lids screws onto the container body.

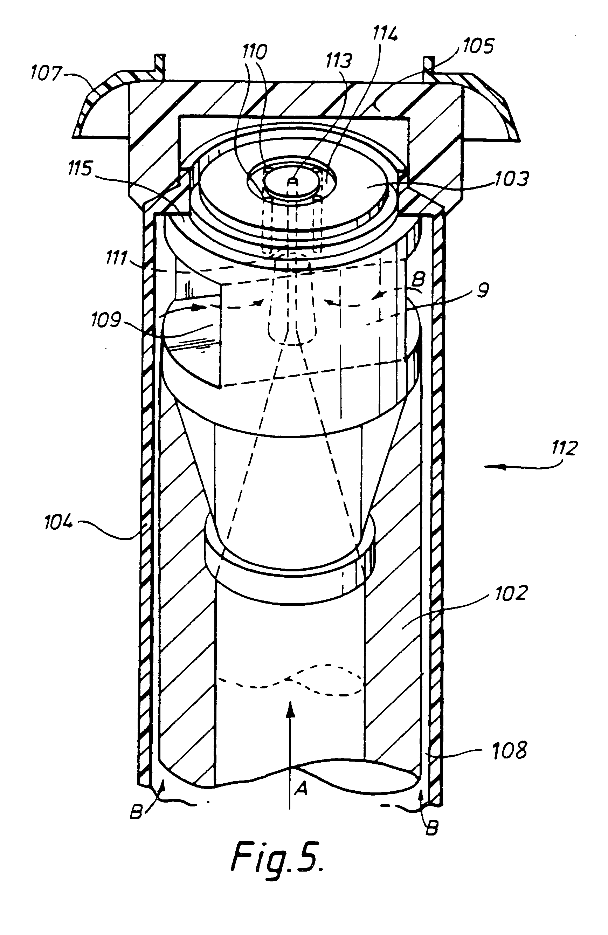

A nozzle head (103) is seated on the top end of the gas duct (102). The gas duct (102) is preferably moulded around the nozzle head (103) so that the head is not removable. The nozzle head (103) has a transverse gallery (109) which extends across the full width of the nozzle head (103). The nozzle head (103) also includes a gas connection tube (111) which leads gas from the gas duct (102) out through the top of the nozzle head (103) to a gas outlet (113). The gas connection passage (111) is visible in Figure 4 as it passes through the transverse gallery (109). In the upper surface of the nozzle head (103) an annular groove is formed which encircles the gas outlet (113). This groove forms a reservoir (114) (See Figures 5, 7, 11 and 12) which, in use, contains a quantity of the substance to be atomised. At least two holes (110) lead from the gallery (109) to the reservoir (114). As pressurized gas leaves the head (103) through the gas outlet (113), the substance contained in the reservoir (114) is atomized into the stream of air. This also causes more of the substance to be drawn into the reservoir (114) from the gallery (109) through the holes (110). The mechanism by which atomization and filling of the reservoir (114) takes place will be explained below. The nozzle head (103) also includes one or more shoulders (115) around the top of the head (103).

A freely rotatable baffle assembly (112) fits over and around the nozzle head (103) and the gas duct (102). The baffle assembly (112) includes a baffle tube (104) which fits around the nozzle head (103) and gas duct (102), a bridge bar (105) which sits above the nozzle head (103), and a mushroom baffle (107) which directs the pressurized air and atomised substance downwards before they can escape through an outlet in the lid (106). The baffle tube (104) slides over the nozzle head (103) and gas duct (102) and reaches almost to the bottom of the container (101). The inside of the baffle tube (104) contains one or more steps (116) positioned to engage with the shoulders (115) of the nozzle head (103). The baffle assembly is thereby supported by the nozzle head (103). The steps (116) seat on the shoulders (115) to form a seal. When seated on the nozzle head (103), the baffle assembly (112) locates the bridge bar (105) directly across the path of the stream of air which passes out from the gas connection tube (111) of the nozzle head (103) and diametrically across the annular reservoir (114).

The mushroom baffle (107) extends outwardly from the baffle tube (104) and slopes towards the container (101) the further it extends from the baffle tube (104). Part of the mushroom baffle (107) projects upwardly and fits into an inlet chimney in the lid (106). If desired, the mushroom baffle may simply abut against the lower end of the inlet chimney. In some circumstances, the droplets or particles which enter the stream of gas are too large, and so the lower surface of the mushroom baffle directs them downwards back into the container. The large droplets gather on the underside of the mushroom baffle before running to the edge and dropping back into the container. As the gas exits from the gas outlet (113) in the top of the nozzle head (103) it is diverted by the bridge bar (105) so that it is directed across the reservoir (114). The airflow through a nebuliser is usually controlled externally. For example, in order to administer a medicine to a patient, the atomised substance must be put into the inhaled air. The patient's inhaled air is directed through the nebuliser before entering the patient. Air (C) is drawn into the nebuliser through the inlet chimney in the lid (106). This air meets the emerging gas from the gas outlet (113). The emerging gas causes atomization of the substance, and these atomised particles are then carried away by the inhaled air (A). The atomised substance is directed downwards by the mushroom baffle (107). The passage of the gas over the reservoir (114) atomises the substance to be atomised, carries the substance away and draws more of the substance into the reservoir (114). The air passes downwards, under the influence of the mushroom baffle. Oversize particles are collected on the underside of the mushroom baffle (107). The air then passes over the upper surface of the mushroom baffle (107) and leaves the atomizer through an outlet in the lid (106).

The lid (106) fits onto the container body (101) and holds the baffle assembly (112) securely in place on the nozzle head (103) thereby forming a seal between the nozzle head and the baffle.

The baffle assembly (112) of the present invention does not require seating against the nozzle in any one angular position. Since the baffle assembly (112) is freely angularly rotatable on the nozzle head (103), it may be seated in any orientation as long as the step (114) is seated on the shoulder (115). The bridge bar is, therefore, freely angularly displaceable in line with the gas outlet. This arrangement is therefore an improvement over the prior art because it is much easier to reassemble, in that it is not necessary to position the baffle assembly in one particular position. The feature which is very important considering the elderly or infirm patients who must use it.

Figure 5 shows a sectional view of the assembled gas duct (102), nozzle head (103) and baffle assembly (112). The gas duct (102) is moulded around the nozzle head (103) to attach the nozzle head to the gas duct (102). The nozzle head (103) is shaped with various external features such as flanges in order that once the gas duct has been moulded around the nozzle head (103), the two may not be separated. The gas (A) passing up the gas duct (102) enters the head and passes through the gas connection tube (111). The gas leaves the head (103) through a gas outlet (113) in the top of the head. Encircling the gas outlet (113) is the annular groove in the top of the head (103). This annular groove forms the reservoir (114). Around the gas outlet (113) and disposed within the reservoir (114) are a number of holes (110), in this case four, but there must be at least two which lead the substance to be atomised from the transverse gallery (109) to the reservoir (114). The holes (110) are equally spaced from each other. The bridge bar (105) of the baffle assembly is disposed across the stream of gas, thereby acting as a gas flow control surface. Because there is a reservoir all the way around the gas outlet, it does not matter in what orientation the bridge bar (105) lies across the gas outlet (113). The stream of gas emerging from the nozzle head (103) is divided by the bridge bar so that it flows over the reservoir. The gas draws the substance to be atomised through the holes (110) thereby filling the reservoir (114). As a result of the substance being drawn through the holes (11), a flow of the substance occurs following arrows B. The substance is held in the container (101) from where it flows between the outer surface of the gas duct (102) and the inner surface of the baffle tube (104). The substance then enters the gallery (109) from each end. Once the gallery (109) is filled with the substance, the substance passes through the holes (110) into the reservoir (114) of the nozzle head (103) and into the stream of gas.

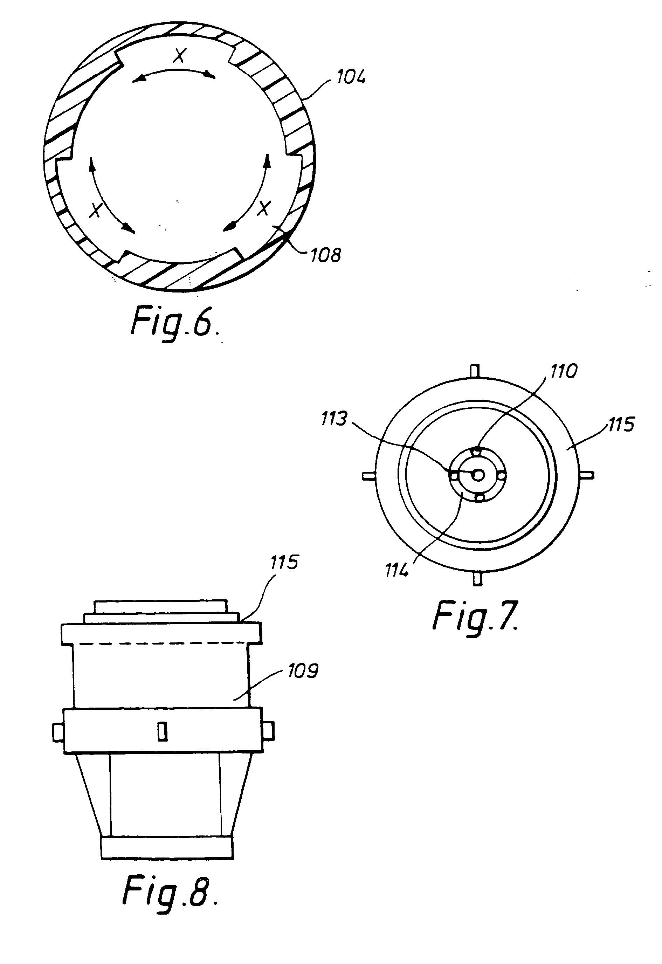

Figure 6 shows a section through the baffle tube (104). Three longitudinal grooves (108) run up the inner surface of the baffle tube (104) forming supply passages up which the substance to be atomised passes. The three grooves (108) are evenly spaced, and all are of the same angular width X. The baffle tube (104) in which the grooves (108) are located is part of the baffle assembly (12). The tube (104) is, therefore, - rotatable about the axis passing through the longitudinal center of the tube (104). It is desirable that the channels formed by the grooves (108) always align with the openings into the transverse galley (109), so that the substance may always pass from the container (101) shown in Figure 4 to the gallery (109) shown in Figure 5. To ensure this, the grooves are wide enough that no matter what angular position the baffle tube (104) is in, the substance to be atomised is always able to enter the gallery Preferably, the substance should always enter the gallery from both ends. If the substance is always to enter the gallery from both ends, the part of the tube (104) between the grooves must be narrower than the width of the gallery (109) at each end.

Figure 7 shows a plan view of the nozzle head. The central hole is the gas outlet (113) of the gas connection passage. Disposed equiangularly around the gas outlet (113) are four holes (110) from which the substance to be atomised is drawn. These holes (110) are disposed in the annular groove which forms the reservoir (114) around the gas outlet (113). The nozzle head also includes a shoulder (115) running around the top of the nozzle head (103) on which the baffle assembly (112) is rotationally seated.

Figure 8 shows the nozzle head (103) before being moulded into the gas duct (102). The gallery (109) is shown by doted lines. The gallery (109) is open at each end. The shoulder (115) may be seen, on which the baffle assembly is seated. This seat preferably forms a seal.

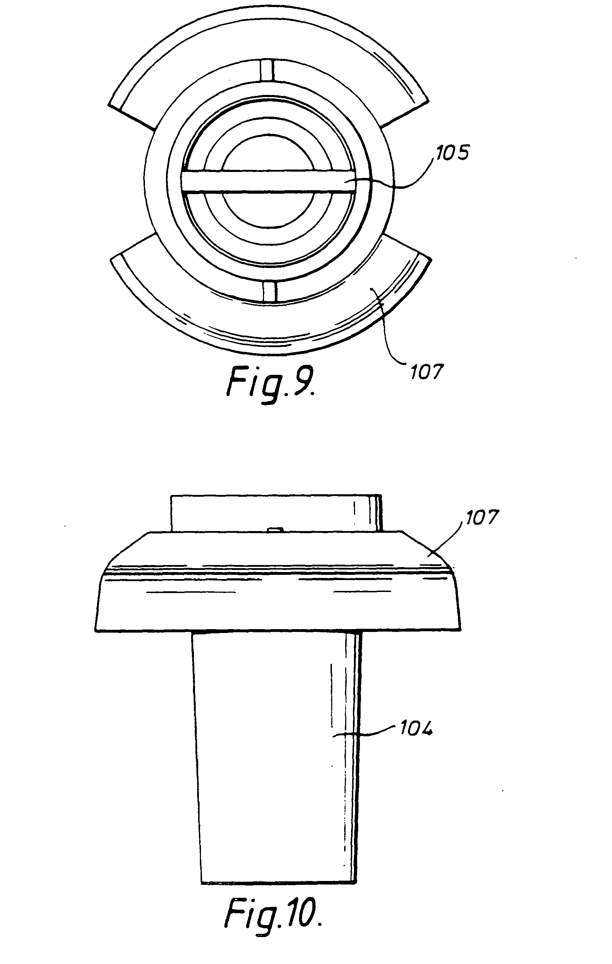

Figure 9 and 10 show a baffle assembly (112) which has a slightly different mushroom baffle (107). Since the stream of pressure gas is divided by the bridge bar (105), two streams of pressure gas pass radially across the reservoir (114) in opposite directions. Both streams of gas are directed downwardly and around the mushroom baffle (107) but at diametrically opposed sides of the mushroom baffle (107). The streams of gas only meet part of the mushroom baffle (107) the rest having no purpose. These sections of the mushroom baffle (107) which do not direct gas downwards may be omitted, as in this embodiment, which assists in atomization and helps to equalise pressure in the region of the bridge bar (105).

In other embodiments of this invention, several variations may be made. For example, the gallery (109) need not be a single gallery (109) across the head, but may be a double or triple gallery. Each gallery would cross the other gallery or galleries forming a cross shaped, or a star shaped gallery with four or six or more open ends to the galleries.

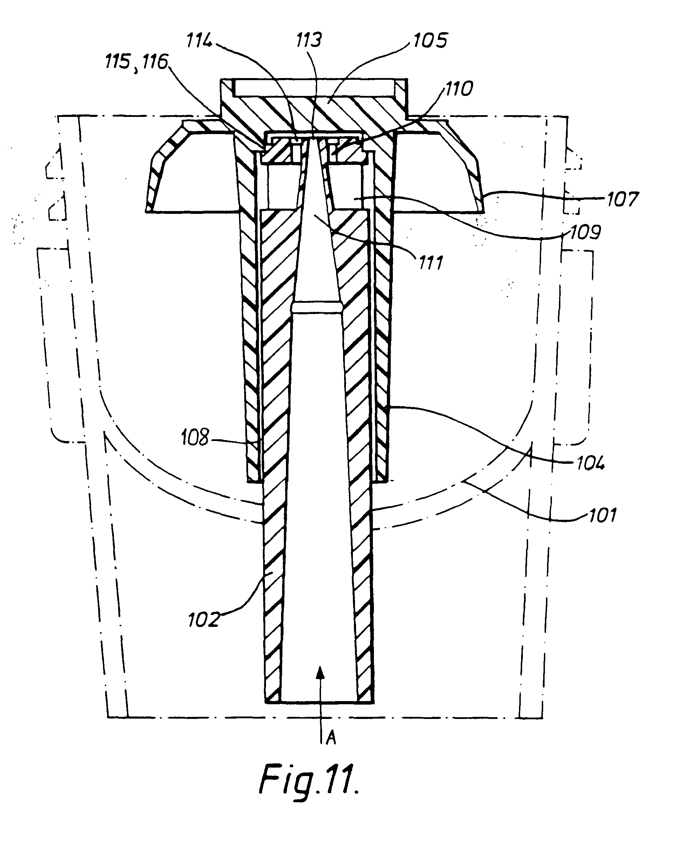

Figure 11 shows the baffle assembly (112) according to the main embodiment of the invention seated on the nozzle head (103). The gas (A) passes up the gas duct (102) and through the gas connection tube (111) in the nozzle head (102). As the gas exits from the top surface of the nozzle head (102) through the gas outlet (113), it is divided by the bridge bar (105). The gas is directed over the reservoir (114) where the substance is atomised. The gas then meets a flow of air which is being inhaled by a patient, and together the air, gas and atomised substance are directed downwards by the lower surface of the mushroom baffle (107) and back up around the mushroom baffle (107) to an outlet in the lid (106). The substance to be atomised is drawn from the container (101), along grooves (108) between the gas duct (102) and the baffle tube (104), - see also Figure 3 and into the transverse gallery (109). From the gallery (109), the substance passes through the holes (110) into the reservoir (114).

The number of grooves corresponding to grooves (108) (see Figure 6) opening into the inner surface of the baffle tube (104) may vary. For example, five grooves may be used and, if a double or triple gallery is used, then five or seven grooves (108) may be much more suitable.

Furthermore, the grooves (108) may be positioned on the outer surface of the gas duct (102) so that the grooves (108) lead directly to the ends of the gallery or galleries. In that situation, the inner surface of the baffle tube (104) may be free of grooves.

In other embodiments, the number of holes (110) surrounding the gas outlet (113) may vary from three upwards. Four has been found to give excellent results.

Furthermore, it may be possible to improve atomization by shaping the holes (110). For example, the holes (110) may run helically through the nozzle head (103) in order to increase turbulence in the flow of the substance to be atomized.

## Claims

1. A nebulizer for atomising a substance into a stream of gas including:
a casing
a nozzle head (103) disposed within said casing and having at least one gas outlet (113) for forming the stream of gas,
means for introducing into the nebulizer the substance to be atomised and
an airflow control device having a bridge bar (105) which is locatable for interception of the stream of gas issuing from said at least one gas outlet (113) for deflecting the stream of gas issuing from said at least one gas outlet,
the nozzle head including an open reservoir (114) for receiving the substance to be atomised and from which the substance is atomised by the stream of gas, at least two holes (110) opening into the open reservoir and through which the substance to be atomised is drawn by the stream of gas issuing from said at least one gas outlet, the bridge bar (105) being freely angularly displaceable in line with said at least one gas outlet for deflecting the stream of gas across the open reservoir, **characterised in that** the open reservoir is defined by an annular groove encircling the said at least one gas outlet.

2. A nebulizer according to claim 1 **characterised in that** the airflow control device includes a tubular carrier (104) which fits on and around the nozzle head (103) to support the bridge bar (105) to be angularly displaceable.

3. A nebulizer according to claim 1 or 2 **characterised in that** it includes a source of the substance to be atomised.

4. A nebulizer according to claim 3 **characterised in that** the source is a container (101) for carrying the substance to be atomised.

5. A nebulizer according to any preceding claim **characterised in that** the nozzle head (103) includes a transverse gallery (109) via which the substance to be atomised is supplied to the said at least two holes (110).

6. A nebulizer according to claim 5 **characterised in that** the transverse gallery (109) opens out of the nozzle head (103) at one or more ends of the gallery.

7. A nebulizer according to claim 2 and any of claims 5 and 6 **characterised in that** the substance to be atomised is drawn into the transverse gallery (109) from between an outer surface of the nozzle head (103) and an inner surface of the tubular carrier (104).

8. A nebulizer according to claim 7 **characterised in that** the nebulizer includes at least one longitudinal groove (108) disposed in at least one of the outer surface of the nozzle head (103) and the inner surface of the tubular carrier (104).

9. A nebulizer according to claim 8 **characterised in that** said at least one longitudinal groove (108) is in alignment with at least one open end of the transverse gallery (109).

10. A nebulizer according to claims 7, 8 or 9 **characterised in that** the nebulizer includes three longitudinal grooves (108).

11. A nebulizer according to claim 8, 9 and 10 **characterised in that** the substance to be atomised is drawn into the transverse gallery (109) from the container (101) via the longitudinal groove means (108).

12. A nebulizer according to claim 2 and any of claims 4 to 11 **characterised in that** the nozzle head (103) is mounted at an end of a gas duct (102) which passes through the container (101), and the tubular carrier (104) extends downwardly around the gas duct to the bottom of the container.

13. A nebulizer according to claims 4 to 12 **characterised in that** the airflow control device includes a mushroom baffle (107) which extends outwardly and downwardly from the region of the nozzle head serving to deflect oversize droplets back into the container (101).

14. A nebulizer according to any preceding claim **characterised in that** the nebulizer includes a secondary airstream inlet and a secondary airstream outlet which permits a secondary airstream to flow through the nebulizer to carry the atomised substance.

15. A nebulizer according to claim 14 **characterised in that** the secondary stream of air is generated by the inhalation of a person.

16. A nebulizer according to any preceding claim **characterised in that** the said at least two holes (110) are curved for inducing turbulence in the substance to be atomised as it is delivered to the reservoir (114).

17. A nebulizer according to any preceding claim **characterised in that** the nebulizer includes at least three holes (110).

18. A nebulizer according to claims 2 - 17 **characterised in that** the tubular carrier (104) includes spacing means for positioning the bridge bar (105) in a spaced relationship relative to the reservoir (114) and said at least one gas outlet (113).

19. A nebulizer according to any one of claims 1 and 3 to 18, **characterised in that** the airflow control device includes a tubular carrier (104) which fits on and around the nozzle head (103) to support the bridge bar (105) to be angularly displaceable, and **in that** the nozzle head (103) includes one or more shoulders around the top of the head and the inside of the tubular carrier (104) contains one or more steps (116) which seat on the shoulders to form a seal.

## Patentansprüche

1. Zerstäuber zum Zerstäuben einer Substanz in einen Gasstrom hinein, umfassend: ein Gehäuse, einen innerhalb dieses Gehäuses angeordneten und mit wenigstens einem Gasauslass (113) versehenen Düsenkopf (103) zur Bildung des Gasstroms, Mittel zum Einleiten der zu zerstäubenden Substanz in den Zerstäuber, und eine Luftstromsteuervorrichtung mit einem Brückensteg (105), der zur Ablenkung des aus dem wenigstens einen Gasauslass ausfließenden Gasstroms derart positionierbar ist, dass er den aus dem wenigstens einen Gasauslass (113) ausfließenden Gasstrom teilt, wobei der Düsenkopf folgendes aufweist: einen offenen Behälter (114) zur Aufnahme der zu zerstäubenden Substanz, aus dem heraus die Zerstäubung der Substanz durch den Gasstrom erfolgt, wenigstens zwei Löcher (110), die in den offenen Behälter hinein münden und durch die hindurch der Gasstrom, der aus dem wenigstens einen Gasauslass austritt, die zu zerstäubende Substanz mit sich führt, wobei der Brückensteg (105) in Ausrichtung mit dem wenigstens einen Gasauslass in einem Winkel frei verschoben bzw. verdreht werden kann, um den Gasstrom über den offenen Behälter hinweg abzulenken, **dadurch gekennzeichnet, dass** der offene Behälter durch eine Ringnut definiert ist, die den wenigstens einen Gasauslass umgibt.

2. Zerstäuber nach Anspruch 1, **dadurch gekennzeichnet, dass** die Luftstromsteuervorrichtung einen rohrförmigen Träger (104) aufweist, der auf und um den Düsenkopf (103) herum passt, um den Brückensteg (105) winklig verschiebbar zu lagern.

3. Zerstäuber nach Anspruch 1 oder 2, **dadurch gekennzeichnet, dass** er eine Quelle der zu zerstäubenden Substanz aufweist.

4. Zerstäuber nach Anspruch 3, **dadurch gekennzeichnet, dass** es sich bei der Quelle um einen Behälter (101) zum Tragen der zu zerstäubenden Substanz handelt.

5. Zerstäuber nach einem der voranstehenden Ansprüche, **dadurch gekennzeichnet, dass** der Düsenkopf (103) eine Quergalerie (109) aufweist, über die die zu zerstäubende Substanz an die wenigstens zwei Löcher (110) zugeführt wird.

6. Zerstäuber nach Anspruch 5, **dadurch gekennzeichnet, dass** sich die Quergalerie (109) an einem oder mehreren Enden der Galerie aus dem Düsenkopf (103) heraus öffnet.

7. Zerstäuber nach Anspruch 2 und einem der Ansprüche 5 und 6, **dadurch gekennzeichnet, dass** die zu zerstäubende Substanz aus dem Zwischenraum zwischen einer äußeren Oberfläche des Düsenkopfes (103) und einer inneren Oberfläche des rohrförmigen Trägers (104) heraus in die Quergalerie (109) hinein gezogen wird.

8. Zerstäuber nach Anspruch 7, **dadurch gekennzeichnet, dass** er wenigstens eine Längsnut (108) aufweist, die in wenigstens einer der äußeren Oberfläche des Düsenkopfes (103) und der inneren Oberfläche des rohrförmigen Trägers (104) vorgesehen ist.

9. Zerstäuber nach Anspruch 8, **dadurch gekennzeichnet, dass** diese wenigstens eine Längsnut (108) mit wenigstens einem offenen Ende der Quergalerie (109) ausgerichtet ist.

10. Zerstäuber nach Anspruch 7, 8 oder 9, **dadurch gekennzeichnet, dass** er drei Längsnuten (108) aufweist.

11. Zerstäuber nach Anspruch 8, 9 und 10, **dadurch gekennzeichnet, dass** die zu zerstäubende Substanz über das Längsnutmittel (108) aus dem Behälter (101) heraus in die Quergalerie (109) hinein gezogen wird.

12. Zerstäuber nach Anspruch 2 und einem der Ansprüche 4 bis 11, **dadurch gekennzeichnet, dass** der Düsenkopf (103) an einem Ende einer durch den Behälter (101) hindurch verlaufenden Gasleitung (102) angebracht ist, und der rohrförmige Träger (104) um die Gasleitung herum nach unten zum Behälterboden hin verläuft.

13. Zerstäuber nach den Ansprüchen 4 bis 12, **dadurch gekennzeichnet, dass** die Luftstromsteuervorrichtung eine pilzkopfförmige Ablenkhaube (107) aufweist, die vom Bereich des Düsenkopfes nach außen und nach unten verläuft und zum Ablenken von zu großen Tröpfchen in den Behälter (101) zurück dient.

14. Zerstäuber nach einem der voranstehenden Ansprüche, **dadurch gekennzeichnet, dass** er einen zweiten Luftstromeinlass sowie einen zweiten Luftstromauslass aufweist, wodurch ein zweiter Luftstrom durch den Zerstäuber fließen kann, um die zerstäubte Substanz mit sich zu führen.

15. Zerstäuber nach Anspruch 14, **dadurch gekennzeichnet, dass** der zweite Luftstrom durch die Inhalation einer Person erzeugt wird.

16. Zerstäuber nach einem der voranstehenden Ansprüche, **dadurch gekennzeichnet, dass** die wenigstens zwei Löcher (110) gekrümmte Form haben, um beim Zuführen der zu zerstäubenden Substanz an den Behälter (114) Turbulenzen in der zu zerstäubenden Substanz zu erzeugen.

17. Zerstäuber nach einem der voranstehenden Ansprüche, **dadurch gekennzeichnet, dass** er wenigstens drei Löcher (110) aufweist.

18. Zerstäuber nach den Ansprüchen 2 bis 17, **dadurch gekennzeichnet, dass** der rohrförmige Träger (104) Abstandsmittel aufweist, die eine Positionierung des Brückenstegs (105) im Abstand zu dem Behälter (114) und dem wenigstens einen Gasauslass (113) ermöglichen.

19. Zerstäuber nach einem der Ansprüche 1 sowie 3 bis 18, **dadurch gekennzeichnet, dass** die Luftstromsteuervorrichtung einen rohrförmigen Träger (104) aufweist, der auf und um den Düsenkopf (103) herum passt, um den Brückensteg (105) winklig verschiebbar zu lagern, und, dass der Düsenkopf (103) an seinem oberen Kopfbereich herum mit einer oder mehreren Schultern versehen ist, und im Inneren des rohrförmigen Trägers (104) eine oder mehrere Stufen (116) vorgesehen ist bzw. sind, die zur Bildung eines Verschlusses auf den Schultern aufsitzen.

## Revendications

1. Nébuliseur pour la pulvérisation d'une substance dans un flux de gaz comprenant
- une enveloppe,
- une tête à buse (103) située à l'intérieur de l'enveloppe et possédant au moins un orifice de sortie de gaz (113) pour former le flux gazeux,
- un moyen d'introduction dans le nébuliseur de la substance à pulvériser et
- un dispositif de commande du flux d'air comportant une barre de pontage (105) pouvant être disposée de façon à intercepter le flux gazeux provenant de cet au moins un orifice de sortie de gaz (113) afin de dévier le flux gazeux provenant dudit au moins un orifice de sortie de gaz,
la tête à buse comprenant un réservoir ouvert (114) pour contenir la substance à pulvériser et d'où la substance est pulvérisée par le flux de gaz, au moins deux trous (110) débouchant dans le réservoir ouvert et à travers lesquels la substance à pulvériser est aspirée par le flux gazeux provenant dudit au moins un orifice de sortie de gaz, la barre de pontage (105) étant librement déplaçable angulairement en ligne avec ledit au moins un orifice de sortie de gaz afin de dévier le flux gazeux à travers le réservoir ouvert, **caractérisé en ce que** le réservoir ouvert est délimité par une gorge concentrique entourant le au moins un orifice de sortie de gaz.

2. Nébuliseur selon la revendication 1 **caractérisé en ce que** le dispositif de commande du flux d'air comporte un support tubulaire (104) qui, assemblé sur et autour de la tête à buse (103), supporte la barre de pontage (105) afin de pouvoir la déplacer angulairement.

3. Nébuliseur selon la revendication 1 ou 2 **caractérisé en ce qu'**il comprend une source de substance à pulvériser.

4. Nébuliseur selon la revendication 3 **caractérisé en ce que** la source est un contenant (101) renfermant la substance à pulvériser.

5. Nébuliseur selon l'une quelconque des revendications précédentes **caractérisé en ce que** la tête à buse (103) comporte un passage transversal (109) par lequel l'alimentation en substance à pulvériser est assurée aux dits au moins deux trous (110).

6. Nébuliseur selon la revendication 5 **caractérisé en ce que** le passage transversal (109) débouche à l'extérieur de la tête à buse (103) à au moins une des extrémités du passage.

7. Nébuliseur selon la revendication 2 et l'une quelconque des revendications 5 ou 6 **caractérisé en ce que** la substance à pulvériser est aspirée dans le passage transversal (109) à partir d'un espace situé entre la surface extérieure de la tête à buse (103) et la surface intérieure du support tubulaire (104).

8. Nébuliseur selon la revendication 7 **caractérisé en ce que** le nébuliseur comprend au moins une rainure longitudinale (108) disposée dans au moins une des surfaces extérieures de la tête à buse (103) et des surfaces intérieures du support tubulaire (104).

9. Nébuliseur selon la revendication 8 **caractérisé en ce qu'**au moins une des rainures longitudinales (108) est alignée avec au moins une des extrémités ouvertes du passage transversal (109).

10. Nébuliseur selon les revendications 7, 8 ou 9 **caractérisé en ce que** le nébuliseur comprend trois rainures longitudinales (108).

11. Nébuliseur selon les revendications 8, 9 et 10 **caractérisé en ce que** la substance à pulvériser est aspirée dans le passage transversal (109) à partir du contenant (101) le long du moyen de rainure longitudinale (108).

12. Nébuliseur selon la revendication 2 et l'une quelconque des revendications 4 à il **caractérisé en ce que** la tête à buse (103) est montée à une extrémité d'un conduit de gaz (102) qui traverse le contenant (101), et **en ce que** le support tubulaire (104) s'étend vers le bas autour du conduit de gaz vers le fond du contenant.

13. Nébuliseur selon les revendications 4 à 12 **caractérisé en ce que** le dispositif de commande du flux d'air comporte un déflecteur (107) en forme de champignon s'étendant vers l'extérieur et vers le bas, servant à dévier les gouttelettes trop grosses vers le contenant (101).

14. Nébuliseur selon l'une quelconque des revendications précédentes **caractérisé en ce que** le nébuliseur comprend un orifice secondaire d'entrée de flux d'air et un orifice secondaire de sortie de flux d'air permettant à un flux d'air secondaire de s'écouler à travers le nébuliseur afin de transporter la substance pulvérisée.

15. Nébuliseur selon la revendication 14 **caractérisé en ce que** le flux d'air secondaire est produit par l'inhalation d'une personne.

16. Nébuliseur selon l'une quelconque des revendications précédentes **caractérisé en ce que** les au moins deux trous (110) sont incurvés afin de provoquer des turbulences dans la substance à pulvériser alors qu'elle est retournée au réservoir (114).

17. Nébuliseur selon l'une quelconque des revendications précédentes **caractérisé en ce que** le nébuliseur comprend au moins trois trous (110).

18. Nébuliseur selon les revendications 2 à 18 **caractérisé en ce que** le support tubulaire (104) comprend un moyen d'écartement afin de positionner la barre de pontage (105) de façon distante du réservoir (114) et dudit au moins un orifice de sortie de gaz (113).

19. Nébuliseur selon l'une quelconque des revendications 1 et 3 à 18, **caractérisé en ce que** le dispositif de commande du flux d'air comprend un support tubulaire (104) ajusté sur et autour de la tête à buse (103) pour permettre à la barre de pontage (105) d'être déplaçable angulairement, et **en ce que** la tête à buse (103) comprend un ou plusieurs épaulements autour du sommet de la tête et que l'intérieur du support tubulaire (104) comprend un ou plusieurs étages qui reposent sur les épaulements pour former une obturation.
